# EUROPÄISCHE PATENTSCHRIFT

(11) **EP 0 500 556 B1**
(45) Veröffentlichungstag und Bekanntmachung des Hinweises auf die Patenterteilung: **19.01.2000**
(21) Anmeldenummer: 90914898.3
(22) Anmeldetag: 12.10.1990
(51) Int. Cl.: A61L 27/00

(54) **WIRKSTOFFKOMPLEX FÜR DIE HERSTELLUNG VON BIOLOGISCHEN TEILEN, INSBESONDERE VON ORGANEN FÜR LEBEWESEN; VERFAHREN ZUM HERSTELLEN DESSELBEN UND SEINE VERWENDUNG**
ACTIVE SUBSTANCE COMPLEX FOR THE PRODUCTION OF BIOLOGICAL PARTS, ESPECIALLY ORGANS FOR LIVING ORGANISMS; PROCESS FOR ITS PRODUCTION AND ITS USE
COMPLEXE DE SUBSTANCE ACTIVE POUR L'OBTENTION D'ELEMENTS BIOLOGIQUES, NOTAMMENT D'ORGANES POUR DES ETRES VIVANTS, SON PROCEDE DE FABRICATION ET SON UTILISATION

(30) Priorität: 03.11.1989 DE 3936568
(43) Veröffentlichungstag der Anmeldung: 02.09.1992
(73) Patentinhaber: SCHMIDT, Karlheinz, Prof. Dr. Dr., 72810 Gomaringen (DE)
(72) Erfinder: SCHMIDT, Karlheinz, Prof. Dr. Dr., 72810 Gomaringen (DE)
(86) Internationale Anmeldenummer: DE9000782
(87) Internationale Veröffentlichungsnummer: WO9106324

(56) Entgegenhaltungen:
- EP-A- 0 271 668
- WO-A-88/07078
- WO-A-90/00060
- FR-A- 2 637 502
- GB-A- 2 137 209

## Beschreibung

Die einzelnen Funktionsleistungen eines Lebewesens (Menschen, Tiere, Pflanzen) sind im allgemeinen nicht diffus über seinen Körper verteilt, sondern nach einem für die jeweilige Art charakteristischen Bau- und Funktionsplan in verschiedene biologische Teile separiert und konzentriert. Diese biologischen Teile, auf die sich auch die Erfindung bezieht, bezeichnet man als Organellen, Zellen, Gewebe- oder Organteile. Vielfach sind diese wiederum zu komplexeren Systemen, auf die sich die Erfindung ebenfalls bezieht, zusammengefaßt. Beispiele für einzelne derartige biologische Teile sind Muskelgewebe, Knorpel, Knochen, Leber, Milz, Haut etc.. Weitere Beispiele für die zusammengefaßten Systeme sind Nervensystem, Gefäßsystem, Skeletsystem, Hormonsystem, Immunsystem etc..

Im Verlauf und ebenso bei der Entstehung des Lebens kann es bei jedem Lebewesen zu Störungen von Struktur und Funktion einzelner biologischer Teile bis hin zum kompletten Funktionsverlust derselben oder ganzer Systeme kommen. Die Ursache hierfür können unter anderem darin begründet sein, daß familiär-genetische bzw. konstitutionelle Störungen vorliegen, daß Mikroorganismen wie Bakterien, Viren, Pilze oder Parasiten die biologischen Teile schädigen oder zerstören, daß Verletzungen durch mechanische, chemische, thermische, elektrische oder radiologische Einwirkungen erfolgt sind oder daß Störungen unbekannter Ursache auf die biologischen Teile einwirken wie verschiedene degenerative Prozesse, Krebs oder manche Arten von Altersveränderungen.

Wirkstoffkomplexe für die Herstellung bestimmter Arten von biologischen Teilen sind bekennt. So wird ein bekannter Wirkstoffkomplex (GB-A-2 137 209) für die Reparatur von Knorpeln und Knochen eingesetzt. Er weist neben einer Strukturkomponente in Form eines Gels gemäß dem Merkmal a) eine Wachstums- und Maturationskomponente in Form eines Cytokins gemäß dem Merkmal e) des Hauptenspruches auf. Dieser Wirkstoffkomplex dient als Implantat und weist entweder embryonale Knorpelzellen auf, die unter dem Einfluß der Wachstums- und Maturationskomponente in Knorpelzellen umgewandelt werden, oder baut Knorpelzellen, die sich in der Umgebung des Implentats befinden, in die Strukturkomponente ein.

Ein weiterer bekannter Wirkstoffkomplex (EP-A-0 271 668) weist eine Strukturkomponente in Form eines Netzwerkes gemäß dem Merkmal b) sowie eine Wachstums- und Maturetionskomponente in Form eines Cytokins gemäß den Merkmal e) des Hauptanspruches auf. Dieser Wirkstoffkomplex wird für Knochendefekte mit Hohlraumbildung und Paradonditis eingesetzt, wobei die Strukturkomponente Knochenzellen aufweist, die von der Wachstums- und Maturetionskomponente unter Bildung von Knochenmaterial zu Wachstum angeregt werden.

Ein zielgerichteter Aufbau beliebig vorgebbarer biologischer Teile, insbesondere von Organen, ist mit diesen beiden bekannten Komplexen nicht möglich.

Es ist ferner eine Immobilisierungssubstenz bekannt (WO-A-88/07078), die über eine unlösliche Adhäsionskomponente gemäß dem Herzmal d.) des Hauptanspruches in Form von Proteinen verfügt, die aus extrazellulären Strukturen, wie Fimbrien, Pili sowie aus Bakterien isolierbar sind. Mittels dieser Immobilisierungssubstanz lassen sich Zellen an Träger in Form von Implantatoberflächen binden, um Abstoßungsreaktionen vermeiden zu helfen.

In einer nachveröffentlichten Druckschrift (FR-A-2 637 502) ist ein Wirkstoffkomplex beschrieben, der eine Strukturkomponente gemäß dem Merkmal b) in Form eines Korallenmaterials, eine unlösliche Adhäsionskomponente gemäß dem Merkmal d) in Form von adhäsiven Proteinen sowie eine Wachstumskomponente gemäß dem Merkmal e) des Hauptanspruches aufweist. Auch dieser Wirkstoffkomplex dient dem Aufbau von Knochenmaterial, wobei die Strukturkomponente in Form der Koralle abgebaut und die eigentliche Knochenstruktur mittels Osteoblasten aufgebaut wird. Hierbei wird durch das Vorhandensein der für den Korallenabbau notwendigen Osteoclasten der Aufbau der Knochenstruktur mittels Osteoblasten gestört. Ein Aufbau von beliebig vorgebbaren biologischen Teilen ist auch mit diesem Komplex nicht erreichbar.

Durch die GB-A 2 215 209 ist ein Device bekannt, das die Merkmale a) bis c) und e) des Hauptanspruches aufweist. Als Rekrutierungskomponente kommt hierbei Fibronectin als löslicher chemotaktischer Stoff zum Einsatz. Mit dem bekannten Device lassen sich Defekte auffüllen und es dient der Wiederherstellung funktioneller oder mechanischer Eigenschaften mit Initiierung von Knochen- und Knorpelbildung etc. Die Herstellung von biologischen Teilen, insbesondere von Organen für Lebewesen ist mit dem bekannten Device nicht möglich.

In der WO 84/00540 ist offenbart, daß Fibronectin nicht nur eine chemotaktische Wirkung ausübt, also als lösliche Rekrutierungskomponente gemäß dem Merkmal c) des Hauptanspruches dient, sondern auch eine Adhäsionskomponente ist. Dahingehende Adhäsine sind aber unlösliche Stoffe, so daß der Einsatz von Fibronectin als lösliche Rekrutierungs- oder als unlösliche Adhäsionskomponente jeweils die gleichzeitige Verwendung als unlösliche Adhäsions- bzw. lösliche Rekrutierungskomponente ausschließt.

Aufgrund dieses Umstandes würde also ein Einsatz des in der WO 84/00540 offenbarten Fibronectins mit den dort beschriebenen Eingenschaften auf ein Device gemäß der Lehre der GB-A 2 215 209 nicht den erfindungsgemäßen Wirkstoffkomplex mit seinen vorteilhaften Eigenschaften ergeben.

Als Stand der Technik gilt es ferner, Störungen oder den kompletten Verlust einzelner biolopischer Teilfunktionen durch Anwendung chemischer Verbindungen auszugleichen zu versuchen. Hierbei soll ein Mangel oder das komplette Fehlen einzelner Stoffwechselprodukte, die durch den jeweiligen biologischen Teil hergestellt werden, kompensiert werden. Ein Beispiel hierfür ist etwa der Ausfall einer Teilfunktion der Bauchspeicheldrüse, der zu einem Insulinmangel oder einem kompletten Fehlen an diesem Syntheseprodukt der Bauchspeicheldrüse führt. Hierbei dient nun von Tieren gewonnenes sowie chemisch oder biotechnisch hergestelltes Insulin zur Kompensation. Für diese Art der Anwendung chemischer Verbindungen zur Kompensation eines Verlustes biologischer Teilfunktionen gibt es zahlreiche Beipiele, man denke etwa an die verschiedensten Hormone oder an Gerinnungsfaktoren, die bei den entsprechenden Störungen die Teilfunktion restituieren sollen. Diese Form der Anwendung chemischer Verbindungen bzw. biologischer Syntheseprodukte kann die ausgefallenen biologischen Teilfunktionen nur partiell kompensieren, solange bei der Anwendung nur zeitweilig wirkende Depots gesetzt werden können und keine, auf den aktuellen Bedarf abgestimmte, also regulierte Freisetzung der Wirkstoffe bzw. Ersatzstoffe im Körper erfolgt.

Aufgrund dieser Probleme und insbesondere bei Auftreten von Störungen mehrerer Teilfunktionen oder bei Zerstörung eines gesamten biologischen Teils gehört es ferner zum Stand der Technik, die oft lebenswichtigen defekten oder verloren gegangenen Teilfunktionen in einen technischen Apparat zu integrieren und diesen an den Körper anzuschließen oder in ihn einzubauen im Sinne einer Prothese. Beispiele für derartige Hilfsmittel in extrakorporaler Anwendung sind etwa Brillen, Hörgeräte, Arm- oder Beinprothesen, künstliche Nieren etc.. Für intrakorporale Anwendung sind Herzschrittmacher, Blutgefäßprothesen, künstliche Gelenke etc. bekannt, und es gibt auch gemischte extra/intrakorporale Hilfsmittel, wie z.B. Zahnimplantate. Anwendungsfähige Prothesen für den intrakorporalen Ersatz innerer Organe wie etwa das Kunstherz, die künstliche Bauspeicheldrüse, die künstliche Leber, oder Niere gehören noch nicht zum Stand der Technik. Grundsätzlich bleibt bei einem technischen Ersatz für biologische Teile festzuhalten, daß nur Teilfunktionen ersetzt werden können und niemals die Gesamtheit der Funktionen des derart ersetzten biologischen Teils. Hinzu kommt, daß die Funktionstüchtigkeit des technischen Systems, also der Prothese bzw. des künstlichen Organs über längere Zeiträume nicht gewährleistbar und eine Anpassung an sich ändernde Bedingungen nicht wie bei einem biologischen Teil möglich ist. Hinzu kommen erhebliche Akzeptanzprobleme, einerseits einen Fremdkörper im Organismus zu tolerieren und andererseits das Gefühl der Abhängigkeit von der Funktion eines technischen Apparates zu haben.

Ferner gehört die Transplantation biologischer Teile zum Stand der Technik. Hierbei ist eine Übertragung biologischer Teile innerhalb desselben Organismus (autogene Transplantation), eine Übertragung von einem Spender derselben Art wie der Empfänger auf diesen (allogene Transplantation) oder eine Übertragung von einem Spender anderer Spezies wie der Empfänger auf diesen (xenogene Transplantation) bekannt.

Die durch die autogene Transplantation eröffneten Möglichkeiten sind meist begrenzt durch die Verfügbarkeit an biologischen Teilen für eine Übertragung. Sie wird aber, wo sie möglich ist, bevorzugt angewandt, da sie keine immunologischen Probleme bereitet. Beispiele hierfür sind Hautverpflanzungen, die Autotransfusion von Blut, die Transplantation von Muskeln, Fettgewebe, Knochen, Knorpel, Blutgefäßen etc..
Aus Gründen der Verfügbarkeit heraus wird daher auf allogene Transplantate zurückgegriffen. In diesem Bereich hat sich etwa die Bluttransfusion trotz der vielen mit ihr verbundenen Probleme in breitem Umfang durchgesetzt, und neben den hierfür vorgesehenen Blutbanken haben sich auch Haut- und Knochenbanken etabliert. Zahlreiche andere Organe werden ebenfalls allogen transplantiert, wie z.B. Niere, Herz, Leber, Pankreas, Knochenmark, Hornhaut etc..
Bei der allogenen und xenogenen Transplantation biologischer Teile ergibt sich jedoch das Problem der immunologischen Inkompatibilität, die zu Abstoßungsreaktionen des Lebewesens gegen das Transplantat führt, die meist zu lebenslanger Einnahme von Immunsuppressiva zwingt bei denen schädliche Nebenwirkungen auftreten. Zudem besteht stets die Gefahr der Übertragung von Krankheiten, da die "lebenden" biologischen Teile nicht sterilisiert werden können. Durch die allogene Transplantation geht darüberhinaus die genetische Einheitlichkeit des Empfängers verloren, der dadurch zur Chimäre wird.

Ausgehend von diesem Stand der Technik liegt der Erfindung die Aufgabe zugrunde, eine Möglichkeit zu schaffen, beliebig vorgebbare biologische Teile eines Lebewesens zu ersetzen, ohne daß die im Stand der Technik bekannten Nachteile auftreten.

Diese Aufgabe löst erfindungsgemäß ein Wirkstoffkomplex mit den Merkmalen des Anspruches 1. Der erfindungsgemäße Wirkstoffkomplex hat die Eigenschaft, mir Zellen in Wechselwirkung zu treten und sie zur Bildung eines biologischen Teils zu veranlassen. Hierzu wird der Wirkstoffkomplex (Implantat), was auch in industriellem Maßstab, beispielsweise im Rahmen einer Serienfertigung vonstatten gehen kann, außerhalb des Körpers des Lebewesens hergestellt und dann mit Zellen in Verbindung gebracht, die das biologische Teil bilden sollen. Dieses kann an einem geeigneten Ort, wo der Wirkstoffkomplex eingesetzt ist, im Körper des Lebewesens selbst erfolgen, aber auch außerhalb des Körpers, beispielsweise in einer Zellkultur. Hierbei wird der erfindungsgemäße Wirkstoffkomplex mit einer Ansammlung vitaler, funktionsfähiger und spezifischer Zellen am gewünschten Ort der Entstehung des biologischen Teils zusammengebracht. Bekanntermaßen bestehen biologische Teile im allgemeinen aus spezifischen Zellen und einem von der Zelle hergestellten extrazellulären Material, doch verfügen nur die zellulären Anteile über eine eigene Stoffwechselaktivität. Da der erfindungsgemäße Wirkstoffkomplex alle für die Herstellung des biologischen Teils erforderlichen Signale vermittelt, ist es nun möglich, die hierfür erforderlichen Zellen mit der gewünschten Geometrie am Ort des Wirkstoffkomplexes anzusammeln, festzuhalten, ihre Zahl zu vermehren und sie im Hinblick auf die gewünschten Funktionen auszureifen. Dadurch, daß der Wirkstoffkomplex bei der Herstellung von biologischen Teilen für jeden erforderlichen Teilschritt die jeweils geeignete Komponente enthält, ist deren Herstellung in ihrer Gesamtheit gewährleistet.

Der erfindungsgemäße Wirkstoffkomplex weist mindestens 4 voneinander verschiedene Komponenten (Strukturkomponente, lösliche Rekrutierungskomponente, unlösliche Adhäsionskomponente, Wachstums- und Maturationskomponente) auf, die stofflich nicht miteinander identisch sind. Bringt man Fibronectin zur Bildung der Rekrutierungskomponente in Lösung, muß die Adhäsionskomponente aus einem anderen von dem Fibronectin verschiedenen Stoff gebildet sein, der nicht löslich ist. Dasselbe gilt im umgekehrten Fall, sofern man Fibronectin in unlöslicher Form für die Adhäsionskomponente einsetzt.

Ferner sind mittels dem erfindungsgemäßen Wirkstoffkomplex körpereigene Zellen zur Herstellung des biologischen Teils verwendbar, so daß die bekannten Schwierigkeiten wie sie bei den sonst üblichen Transplantationen auftreten, entfallen. Insbesondere ist keine Krankheitsübertragung möglich, ebenso keine Langzeit-Immunsuppression mit ihren gravierenden Nebenwirkungen erforderlich, und das Individuum bleibt genetisch einheitlich.

Biologische Teile nehmen für ihre Funktionsleistungen einen gewissen Raum ein. Nicht selten ist ihre Funktion an eine bestimmte Geometrie gebunden, und die biologischen Teile weisen bestimmte Grenzen auf, innerhalb derer sie ihre Funktion erfüllen. Dies gilt in gleicher Weise für die mittels des Wirkstoffkomplexes nach Anspruch 1 hergestellten biologischen Teile. Der für die Herstellung verwendete Wirkstoffkomplex erfüllt diese Funktion mit Hilfe einer Strukturkomponente, die einerseits die Platzhalterfunktion ausübt und es andererseits erlaubt, eine geometrische Form vorzugeben, innerhalb derer das hergestellte biologische Teil seine Funktion erfüllt.

Bei einer bevorzugten Ausführungsform der Strukturkomponente des Wirkstoffkomplexes gemäß dem Anspruch 2 handelt es sich vorwiegend um eine makromolekulare dreidimensionale Matrix, die zusammen mit Wasser und Salz in der Form eines Gels unterschiedlichen Quellungszustandes vorliegen kann. So kommen zum Beispiel Proteoglycangele als Matrix in Frage. Auch ein Netzwerk von Fasern, wie z.B. verschiedene Arten von Kollagenen oder Elastin können die Strukturkomponente bilden. Ebenso eignen sich Kombinationen von Gelen mit eingelagerten Fasern im Sinne von Verbundwerkstoffen. Für die unterschiedlichen Anwendungen zur Herstellung biologischer Teile wird die Strukturkomponente unterschiedlich konfektioniert, um beispielsweise als Vlies, als Gelkörper oder Flüssiggel schneidbar, fräsbar, plastisch verformbar oder gießbar anwendbar zu sein.

Die Strukturkomponente wird an die Erfordernisse des herzustellenden biologischen Teils angepaßt, da eine gewisse Spezifität zwischen den zellulären und den extrazellulären Anteilen biologischer Teile besteht. Quellen für die Herstellung der Strukturkomponente sind daher vorwiegend die extrazellulären Materialien verschiedener Gewebe oder Organe, d.h., z.B. für die Herstellung von Haut werden für die Produktion der Strukturkomponente kutane Proteoglycane und Faserproteine eingesetzt, für die Herstellung der Milz die milzspezifischen Proteoglycane und Faserproteine, für die Herstellung von Knochen knochenspezifische Proteoglycane und Faserproteine etc.. Die Strukturkomponete kann auch metallische , keramische, glasartige, polymere oder fetthaltige Trägermaterialien aufweisen, mit deren Hilfe die geometrischen, mechanischen, chemischen oder sonstigen Eigenschaften der Strukturkomponente modifizierbar sind. Dabei kann das Trägermaterial zusammen mit der Strukturkomponente in solider, poröser, membranöser, mizellarer, viskoser oder flüssiger Form vorliegen je nach den Anforderungen, die sich für die Herstellung des biologischen Teils und seine spätere Funktion ergeben.

Bei einer weiteren bevorzugten Ausführungsform des erfindungsgemäßen Wirkstoffkomplexes entfaltet dieser seine Wirkung zur Herstellung der biologischen Teile im wesentlichen nur temporär, d.h., der Wirkstoffkomplex ist derart ausgebildet, daß er zeitlich steuerbar abbaubar und nach der Herstellung des biologischen Teils nicht mehr vorhanden ist. Die Abbaugeschwindigkeit des Wirkstoffkomplexes ist dabei durch unterschiedliche Quervernetzung der polymeren Matrix und/oder den Zusatz von (Enzym)inhibitoren und/oder immunsuppressiven und/oder entzündungshemmenden Stoffe vorgebbar. Bei den hier angesprochenen Inhibitoren kann es sich um niedermolekulare Verbindungen handeln, die das aktive Zentrum des abbauenden Enzyms besetzen, es kann sich aber auch um Komplexbildner handeln, die einen essentiellen Kofaktor des Enzyms an sich binden oder um neutralisierende Antikörper. Weitere Inhibitionemechanismen sind möglich.

Als entzündungshemmende und/oder immunsuppressive Zusätze sind hier verwendbar: Hemmstoffe der Phospholipase, wie beispielsweise Steroide, Hemmstoffe der Cyclooxygenase, wie beispielsweise Indomethacin, Hemmstoffe der Lipoxygenase, wie beispielsweise Nordihydroguaretsäure, Immunsuppressiva vom Typ der Cyclosporine und/oder vom Typ des Antithymocyten-Globulins etc..

Im Rahmen der gestellten Aufgabe, biologische Teile herzustellen, sind lebende Zellen der gewünschten Art im Bereich der Strukturkomponente anzusammeln. Hierfür enthält die Strukturkomponente des Wirkstoffkomplexes einen oder mehrere Rekrutierungskomponenten mit deren Hilfe die gewünschten Zellen zu gerichteter Bewegung angeregt werden. Als Rekrutierungskomponente(n) sind Chemotaktika (Chemotaxine) geeignet.

Die jeweils geeigneten Chemotaktika sind für eine Vielzahl von Zellen beschrieben worden und lassen sich aus humanen, animalischen, pflanzlichen oder mikrobiellen Quellen isolieren oder auch durch chemische Synthese oder biotechnische Verfahren herstellen. Wird die außerhalb des Körpers des Lebewesens hergestellte Strukturkomponente mit deren Rekrutierungskomponente(n) in einen Organismus eingebracht und/oder außerhalb desselben mit Zielzellen in Verbindung gebracht, so baut sie einen Konzentrationsgradienten auf, in dem sich die Zielzellen orientieren, wobei die jeweilige Rekrutierungskomponente mit den spezifischen Erkennungsstrukturen auf den Zielzellen, die auch als Receptoren bezeichnet werden, in Wechselwirkung tritt. Für den Fall, daß das herzustellende biologische Teil aus mehreren Zellsorten zusammengesetzt ist, enthält die Strukturkomponente entsprechend der Anzahl der Zellarten mehrere Rekrutierungskomponenten in Form von Chemotaktika.

Die Spezifität der jeweiligen Rekrutierungskomponente für die verschiedenen Zielzellen sowie das Ausmaß der chemotaktischen Aktivität wird anhand von Untersuchungen ermittelt, bei denen die gerichtete Wanderung der gewünschten Zellen durch definierte Filterporen unter der Wirkung eines Gradienten des Chemotaktikums in einer Kammer gemessen wird. Mittels derartiger Untersuchungstechniken ist das Wirkstoffsystem hinsichtlich seiner jeweiligen Rekrutierungskomponente biologisch standardisierbar, was für die industrielle Herstellung des Wirkstoffkomplexes von Bedeutung ist.

Als Chemotaktika dienen beispielsweise Peptide wie N-F-Met-Leu-Phe und/oder beispielsweise Metabolite der Arachidonsäure, wie Leukotriene, mit deren Hilfe bestimmte Zellen aus dem Blut bzw. Phagocyten anlockbar sind. Proteine, wie beispielsweise ein Mesenchymzellen anlockendes Protein, wirken speziell auf Bindegewebszellen chemotaktisch.

Neben der Spezifität der jeweiligen Rekrutierungskomponente für die gewünschten Zielzellen und dem Ausmaß der chemotaktischen Aktivität ist auch die Zeitdauer über die sich der chemotaktische Konzentrationsgradient aufbaut und erhält eine zu beachtende Größe. Die Anpassung dieser Kinetik an die Erfordernisse zur Herstellung der biologischen Teile wird bei dem erfindungsgemäßen Wirkstoffkomplex durch eine steuerbare Freisetzung der jeweiligen Rekrutierungskomponente aus der Strukturkomponente erreicht. Hierbei spielt nun die Abbaugeschwindigkeit der Strukturkomponente selbst eine Rolle sowie die Art der Verbindung zwischen Strukturkomponente und der jeweiligen Rekrutierungskomponente, z.B. ob es sich um eine kovalente oder um eine assoziative Bindung handelt. Bei kovalenter Bindung wird ein langsamerer Aufbau und eine längere Erhaltung des chemotaktischen Gradienten ereicht als bei einer lediglich assoziativen Bindung durch ionische Kräfte oder Wasserstoffbrückenbindung. Die Rekrutierung der Zellen zur Herstellung des biologischen Teils verläuft aber meist rascher als der Abbau der Strukturkomponente, da die eingewanderten Zellen ganz wesentlich zum Abbau des Proteoglycan/Collagen Werkstoffes beitragen.

Für die Herstellung der biologischen Teile sind nach der Einwanderung der Zellen in die Strukturkomponente diese am Ort derselben zu fixieren, um ihre Auswanderung in die Umgebung zu verhindern und eine stabile Architektur des hergestellten biologischen Teils zu gewährleisten. Hierfür enthält der Wirkstoffkomplex eine oder mehrere Adhäsionskomponenten, mittels der oder denen die eingewanderten Zellen am Ort der Strukturkomponente fixierbar sind. Dabei "verankert" sich die Adhäsionskomponente einerseits an den die biologischen Teile aufbauenden Zellen und andererseits am makromolekularen Netzwerk der Strukturkomponente. Derartige Adhäsine sind bekannt und weisen eine gewisse Verankerungsspezifität auf. Als Beispiele seien Eiweißkörper vom Typ des Fibronectins oder des Laminins genannt, mit deren Hilfe, beispielsweise Bindegewebszellen bzw. Epithelzellen an der Strukturkomponente verankerbar sind. Zahlreiche weitere Adhäsionsfaktoren unterschiedlicher Spezifität sind verfügbar und kommen je nach dem herzustellenden biologischen Teil bei dem erfindungsgemäßen Wirkstoffkomplex zum Einsatz. Hierzu gehören unter anderem die Zell Adhäsions Moleküle L-CAM, N-CAM; die Matrix Adhäsions Moleküle Cytotactin, Tenascin, Laminin, Fibronectin, Collagen Typ IV,V,VII, sowie synthetische Peptide, die Teilsequenzen verschiedener Adhäsine darstellen und Transmembran Verbindungsproteine, wie beispielsweise Integrin.

Um bei der Herstellung der biologischen Teile die Spezifizät der Anheftung der gewünschten Zellen an die Strukturkomponente zu erhöhen, können Antikörper gegen unerwünschte Adhäsionskomponenten eingesetzt werden. Die biologische Aktivität der Adhäsionskomponenten kann in Adhäsionstests verschiedener Art (z.B. mittels Zentrifugalkräften etc.) gemessen werden und sind damit für den gesamten Wirkstoffkomplex standardisierbar.

Häufig sind die zur Herstellung des biologischen Teils in den Bereich der Strukturkomponente chemotaktisch angelockten und durch die geeigneten Adhäsine fixierten Zellen von ihrer Zahl her nicht ausreichend, um das biologische Teil zu konstituieren. Außerdem befinden sich die für diese Prozesse in einem Organismus verfügbaren mobilen Zellen meist nicht in einem hinreichend ausgereiften Zustand um alle Funktionen eines biologischen Teils zu erfüllen, sie stellen vielmehr häufig Vorläufer- oder Stammzellen dar, aus denen sich die funktionsfähigen, reifen Zellen des herzustellenden biologischen Teils erst entwickeln müssen. Hierfür weist der erfindungsgemäße Wirkstoffkomplex mindestens eine Wachstums- und/oder Maturationskomponente auf, vorzugsweise in Form eines oder mehrerer Cytokine, unter dessen bzw. deren Einwirkung die Zahl der eingewanderten Zellen vermehrt wird und andererseits eine Ausreifung der Zellen erfolgt -

Cytokine sind Stoffe unterschiedlicher chemischer Struktur, die dadurch gekennzeichnet sind, daß sie mit Zellen in Wechselwirkung treten und deren Teilungs- und Wachstumsverhalten ebenso wie ihre Ausreifung und Biosyntheseleistung beeinflussen. Cytokine haben damit eine hormonähnliche Wirkung, entfalten diese aber im Gegensatz zu den Hormonen nicht in Fernwirkung sondern in eher lokalisierten Bereichen, was von Vorteil ist bei der Herstellung biologischer Teile, da es sich hierbei ebenfalls um einen lokalisierten Prozess handelt.

Eine Vielzahl verschiedener Cytokine unterschiedlicher Spezifität ist dem Fachmann bekannt. Diese sind zur Beeinflussung von Zellwachstum, Differenzierung und Maturation (Ausreifung) sowie zur Beeinflussung des Stoffwechsels der eingewanderten Zellen als weitere Komponente im erfindungsgemäßen Wirkstoffsystem einsetzbar. Die Spezifität der Cytokine für bestimmte Zellen ist durch die Anwesenheit der entsprechenden Receptoren auf den Zielzellen determiniert, wobei die Interaktion eines Cytokins mit dem Receptor die zellulären Folgeprozesse auslöst. Bei den hier angesprochenen Receptoren auf den Zielzellen handelt es sich um Membranproteine, die mit dem eingesetzten Chemotaktikum in Wechselwirkung treten, es binden und ins Zellinnere einschleusen. Durch ein Recycling der Rezeptoren stehen sie immer wieder für die Bindung von Chemotaktikum zur Verfügung.

Analog ist es mit den Receptoren für die jeweils eingesetzten Cytokine, so daß es sich nur um eine andere Spezifität handelt bei analogem Mechanismus der Wechselwirkung. Während die Bindung des Chemotaktikums zu gerichteter Bewegung der Zielzellen führt, resultiert die Bindung der Cytokine an den entsprechenden Receptor der Zielzelle in Wachstum und/oder Differenzierung. Vielfach sind die Receptoren molekular noch nicht charakterisiert, so daß sie nur an Hand ihrer Spezifität für den betreffenden Liganden (Chemotaktikum, Cytokin etc) erkannt werden.
Dabei ist zu berücksichtigen, daß nicht selten stimulierende oder hemmende Folgeprozesse an den Zellen ausgelöst werden können, je nach der Spezifität von eingesetztem Cytokin und Zielzelle. Der für die Herstellung biologischer Teile gewünschte zelluläre Folgeprozess der Cytokin-Wechselwirkung ist meist an eine duale Signalvermittlung gebunden, so daß in vorteilhafter Weise mindestens zwei Cytokine bei dem erfindungsgemäßen Wirkstoffkomplex verwendet sind, um Wachstum und Differenzierung zu erreichen. Viele Zellen produzieren nach der Interaktion mit einem Cytokin weitere Cytokine und setzen diese frei, wobei sie sich damit selbst stimulieren oder hemmen können (sogenannter autokriner Mechanismus). Nicht selten ändert sich auch mit einzelnen Differenzierungsschritten die Spezifität der Zellen für bestimmte Cytokine, so daß keine Interaktion mehr zustande kommt, oder auch die Wechselwirkung von einem stimulierenden in einen hemmenden zellulären Folgeprozess umschlagen kann. Die Eigenschaften einer Vielzahl von Cytokinen ist bekannt, so daß die Cytokinwirkung im Wirkstoffsystem ebenfalls standardisierbar ist.

Beispiele für Cytokine sind etwa bei der Herstellung von Blut die Kolonie stimulierenden Faktoren, bei der Herstellung von Bindegewebe der Fibroblasten Wachstumsfaktor, bei der Herstellung von Haut der epidermale Wachstumsfaktor, bei der Herstellung von Knorpel der Knorpel induzierende Faktor, bei der Herstellung der Milz oder der Lymphknoten der Lymphocyten aktivierende Faktor sowie Milzpeptide, für die Herstellung von Thymus der T-Zellen Wachstumsfaktor sowie Thymuspeptide, für die Herstellung von Knochen der Knochen Wachstumsfaktor sowie der transformierende Wachstumsfaktor, für die Herstellung von Blutgefäßen der Angiogenese Faktor. Ferner finden noch die folgenden Cytokine Verwendung: Interleukine, Insulin ähnliche Wachstumsfaktoren, Tumor Nekrose Faktor, Prostaglandine, Leukotriene, transformierende Wachstumsfaktoren, von Thrombocyten abstammender Wachstumsfaktor, Interferone sowie von Endothelzellen abstammender Wachstumsfaktor.

Da biologische Teile vielfach aus mehreren Zelltypen zusammengesetzt sind, können Kombinationen vorkommen. So ist z.B. die Entstehung von Blutgefäßen für die Versorgung des hergestellten biologischen Teils wichtig, so daß eine beschleunigte Gefäßenstehung durch Zusatz von Angiogenese Faktor als Cytokinkomponente des Wirkstoffsystems in Frage kommt. Ähnlich kann die beschleunigte Bildung von Nervenverbindungen wichtig sein, was sich durch einen entsprechenden Einsatz von zusätzlichen Cytokinen im Wirkstoffkomplex verwirklichen läßt.

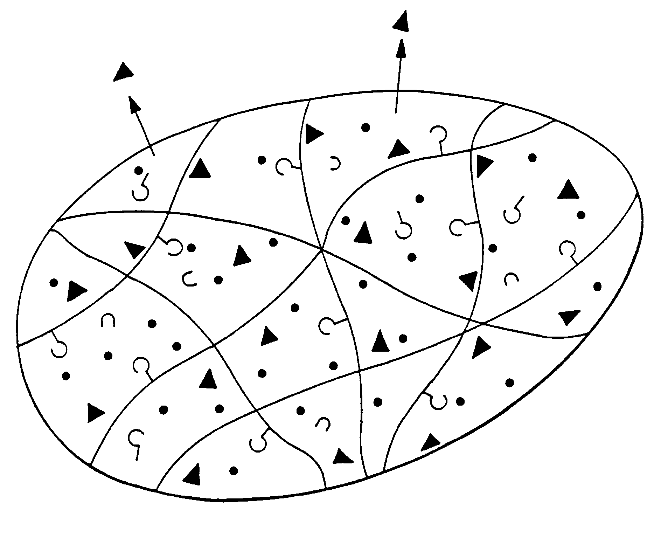

Aus der Figur ist stark vereinfacht der prinzipielle Aufbau des Wirkstoffkomplexes zur Herstellung biologischer Teile ersichtlich. Dabei stellen die durchgehend dargestellten Linien das Netzwerk aus Faserproteinen dar, das zusammen mit dem punktförmig dargestellten Proteoglycangel die Strukturkomponente des erfindungsgemäßen Wirkstoffkomplexes bildet. Die Dreiecke stellen symbolhaft die Rekrutierungskomponente in Form eines Chemotaktikums dar, das , wie das die beiden Pfeile zum Ausduck bringen, teilweise aus dem Wirkstoffkomplex austritt. Die hakenförmigen Teile sind das Symbol für die Adhäsionskomponente und die C-förmigen Klammern für die Wachstums- und Maturationskomponente (Cytokine), die für den Aufbau der gewünschten Zielzellen zum biologischen Teil und zur Unterstützung einer beschleunigten Gefäß- und Nervenversorgung dienen.

Im folgenden wird die Erfindung anhand von zwei Ausführungsbeispielen näher erläutert.

### Ausführungsbeispiel 1: Herstellung eines biologischen Teiles in Form von Haut (Organogenese von Haut)

Humane oder tierische Haut wird mechanisch von den epidermalen Anteilen befreit. Die erhaltene Cutis wird in der sechsfachen Menge (w:v) Chloroform/Methanol 1 : 1 entfettet, in flüssigem Stickstoff tiefgefroren und in einer Gefriermühle bis zu einer Partikelgröße von 400-1000 µm gemahlen. Die Hautpartikel werden anschließend im Hochvakuum (0.05 Torr) unter Kühlung getrocknet, in einer 4 M wässrigen Lösung von Guanidiniumchlorid suspendiert und 12 h bei Zimmertemperatur gerührt. Die unlöslichen Anteile werden verworfen und der lösliche Anteil gegen Wasser erschöpfend dialysiert. Das ausgefällte makromolekulare Netzwerk von Proteoglycanen und Hautkollagen wird gewaschen und im Hochvakuum in der Kälte getrocknet. Das erhaltene Vlies stellt die erfindungsgemäße Strukturkomponente des Wirkstoffsystems zur Auslösung der sogenannten Organogenese von Haut dar, in die anschließend die Adhäsionskomponente, die chemotaktische Komponente (Rekrutierungskomponente), die Wachstums- und Maturationskomponente (Cytokine) eingebracht wird. Als Adhäsionskomponente wird für die Organogenese der Haut Fibronektin in die Strukturkomponente eingearbeitet. Dies geschieht durch Präzipitation, z.B. mittels Äthylalkohol aus wässriger Lösung in Gegenwart der Strukturkomponente oder durch gemeinsame Lyophilisation. Gleichzeitig werden die anderen Komponenten nach demselben Verfahren in die Strukturkomponente eindotiert. Für die kutane Organogenese wird dabei ein Fibrolastenwachstumsfaktor (FGF) verwendet sowie epidermaler Wachstumsfaktor, um das Aufwachsen der Epidermis zu erleichtern. Die Oberfläche wird im Falle der Haut mit einem zusätzlichen Adhäsionsfaktor versehen, um die Anheftung von Epithelzellen zu erleichtern. Dabei handelt es sich um das Laminin, das spezifisch die Anheftung von Epithelzellen erlaubt. Das so hergestellte Wirkstoffsystem wird mit einem Immunsuppressivum zusammen zur Organogenese von Haut, beispielsweise zur Regeneration von Hautdefekten eingesetzt.

### Ausführungsbeispiel 2: Herstellung eines biologischen Teiles in Form von Knochen (Organogenese von Knochen)

Humaner oder tierischer Knochen wird von Weichteilen befreit, die Kondylen werden abgetrennt und die diaphysären Anteile längs aufgesägt und vom Knochenmark befreit. Die erhaltenen Knochenstücke werden in der sechsfachen Menge (w:v) Chloroform/Methanol (1:1) entfettet, in flüssigem Stickstoff tiefgefroren und in einer Gefriermühle bis zu einer Partikelgröße von 400-1000 µm gemahlen. Die Partikel werden anschließend in 0.5 N Salzsäure demineralisiert und danach mit Wasser bis zur Neutralität gewaschen sowie im Hochvakuum unter Kühlung getrocknet.
Die erhaltenen Knochenpartikel werden in einer 4 M wässrigen Guanidiniumchlorid Lösung suspendiert und 12 Stunden bei Zimmertemperatur gerührt. Die unlöslichen Anteile werden verworfen und der lösliche Anteil gegen Wasser erschöpfend dialysiert. Des ausgefällte makromolekulare Netzwerk von Proteoglycanen und Knochenkollagen wird in Wasser gewaschen und im Hochvakuum getrocknet. Das erhaltene Vlies stellt die erfindungsgemäße Strukturkomponente des Wirkstoffsystems zur Auslösung der Organogenese des Knochens dar, in die anschließend die Adhäsionskomponente, die Rekrutierungskomponente, die Wachstums- und Maturationskomponente (Cytokine) eingebracht werden.
Als Adhäsionskomponente dient ein Adhäsin, das aus der Guanidiniumchloridlöslichen Fraktion gewonnen wird. Das Adhäsin erlaubt die Anheftung von Osteoblasten und ihren Vorläuferzellen an die Strukturkomponente. Die Rekrutierungskomponente ist ein Protein, das ebenfalls aus Knochen isoliert wird, wobei die aktive Komponente durch Boyden-Kammer Experimente identifiziert wird. Adhäsionskomponente und Rekrutierungskomponente bilden mit der Strukturkomponente einen Komplex, der die Anlockung und Einnistung von knochenbildenden Zellen bewerkstelligt. Der Komplex wird durch Präzipitation oder Lyophilisation der Komponenten hergestellt.
Zur dualen Vermittlung des Wachstums- und Maturationssignals werden basischer Fibroblasten Wachstumsfaktor und Transformierender Wachstumsfaktor-beta in den Wirkstoffkomplex eindotiert. Zur Wirkungsverstärkung kann dem knochenbildenden Wirkstoffkomplex ein Antikörper gegen katabole Cytokine, wie z.B. Kachexin beigegeben werden. Eine geeignete Konzentration an den Cytokinen im Wirkstoffsystem liegt, z.B. in der Größenordnung von 10ppm.

Die vorstehende Beschreibung und die Zeichnung beschränken sich nur auf die Angabe von Merkmalen, die für die beispielsweise Verkörperung der Erfindung wesentlich sind. Soweit daher Merkmale in der Beschreibung und in der Zeichnung offenbart und in den Ansprüchen nicht genannt sind, dienen sie erforderlichenfalls auch zur Bestimmung des Gegenstandes der Erfindung.

## Patentansprüche (Patentansprüche für folgende(n) Vertragsstaat(en): AT, BE, CH, LI, DE, DU, FR, GB, IT, LU, NL, SE)

1. Wirkstoffkomplex für die Herstellung von biologischen Teilen, insbesondere von Organen für Lebewesen, mit den folgenden, voneinander unterschiedlichen und auf das jeweils herzustellende biologische Teil spezifisch abgestimmten Komponenten in Form
- mindestens einer Strukturkomponente auf der Basis von auf die Zellen des jeweils herzustellenden biologischen Teils spezifisch abgestimmtem extrazellulärem Material,
- mindestens einer Rekrutierungskomponente,
- mindestens einer Adhäsionskomponente,
- mindestens einer Wachstums- und/oder Maturationskomponente, vorzugsweise in Form mindestens eines Cytokins.

2. Wirkstoffkomplex nach Anspruch 1, dadurch gekennzeichnet, daß die Strukturkomponente ein makromolekulares Gel und/oder Netzwerk, vorzugsweise aus Proteoglycanen und Collagenen ist.

3. Wirkstoffkomplex nach Anspruch 1 oder 2, dadurch gekennzeichnet, daß die Strukturkomponente metallische und/oder keramische und/oder glasartige und/oder polymere und/oder fetthaltige Trägermaterialien in solider und/oder poröser und/oder membranöser und/oder mizellarer und/oder plastisch verformbarer und/oder viskoser und/oder flüssiger Form aufweist.

4. Wirkstoffkomplex nach einem der Ansprüche 1 bis 3, dadurch gekennzeichnet, daß die jeweilige Rekrutierungskomponente zur Gruppe der chemotaktischen Peptide, Proteine und/oder der Arachidonsäuremetabolite gehört oder eine andere gleichwirkende chemische Struktur aufweist.

5. Wirkstoffkomplex nach einem der Ansprüche 1 bis 4, dadurch gekennzeichnet, daß die jeweilige Adhäsionskomponente ein Protein vom Typ des Fibronectin, Tenascin, Cytotactin, Laminin, Chondronectin, Collagen Typ IV, V, VII, N-CAM, L-CAM oder Integrin, eine Kombination dieser Proteine oder ein anderes gleichwirkendes Adhäsin ist.

6. Wirkstoffkomplex nach einem der Ansprüche 1 bis 5, dadurch gekennzeichnet, daß die jeweilige Wachstums- und/oder Maturationskomponente zur Gruppe der Kolonie stimulierenden Faktoren (CSF), der Interleukine (IL), der Insulin ähnlichen Wachstumsfaktoren (IGF), der Prostaglandine (PG), der Leukotriene (LT), der Transformierenden Wachstumsfaktoren (TGF), der Fibroblasten Wachstumsfaktoren (FGF), der Interferone (IFN), der epidermalen Wachstumsfaktoren (EGF), der von Knochen abstammenden Wachstumsfaktoren (BDGF), der von Endothelien abstammenden Wachstumsfaktoren (EDGF), der von Thrombocyten abstammenden Wachstumsfaktoren (PDGF) gehört, eine Kombination derartiger Faktoren oder ein anderes gleichwirkendes Cytokin ist.

7. Wirkstoffkomplex nach einem der Ansprüche 1 bis 6, dadurch gekennzeichnet, daß die jeweilige Wachstums- und/oder Maturationskomponente Faktoren zum beschleunigten Einwachsen von Blutgefäßen, wie Angiogenese-Faktoren und/oder von Nerven, wie Nerven-Wachstums-Faktoren, in das herzustellende biologische Teil aufweist.

8. Wirkstoffkomplex nach einem der Ansprüche 1 bis 7, dadurch gekennzeichnet, daß dessen Abbaugeschwindigkeit durch den unterschiedlichen Vernetzungsgrad der Strukturkomponente selbst und/oder den Zusatz von (Enzym)inhibitoren und/oder immunsuppressiven und/oder entzündungshemmenden Wirkstoffen steuerbar ist.

9. Wirkstoffkomplex nach einem der Ansprüche 1 bis 8, dadurch gekennzeichnet, daß dieser zusammen mit Zielzellen ein biologisches Teil, zumindest im Aufbaustadium desselben, bildet.

10. Verfahren zum Herstellen des Wirkstoffkomplexes nach einem der Ansprüche 1 bis 9, dadurch gekennzeichnet, daß dieser durch Kopräzipitation der verschiedenen Komponenten oder durch Lyophilisation oder durch kovalente Verknüpfung der einzelnen Komponenten miteinander herstellbar ist.

11. Verwendung des Wirkstoffkomplexes nach einem der Ansprüche 1 bis 9 zur Herstellung eines biologischen Teils zur Behandlung von Krankheiten, die durch das Fehlen des biologischen Teils oder dessen Fehlfunktion bedingt sind.

## Patentansprüche (Patentansprüche für folgende(n) Vertragsstaat(en): GR, ES)

1. Verfahren zur Herstellung eines Wirkstoffkomplexes für die Herstellung von biologischen Teilen, insbesondere von Organen für Lebewesen, der mit den folgenden, voneinander unterschiedlichen und auf das jeweils herzustellende biologische Teil spezifisch abgestimmten Komponenten in Form
- mindestens einer Strukturkomponente auf der Basis von auf die Zellen des jeweils herzustellenden biologischen Teils spezifisch abgestimmtem extrazellulärem Material,
- mindestens einer Rekrutierungskomponente,
- mindestens einer Adhäsionskomponente,
- mindestens einer Wachstums- und/oder Maturationskomponente, vorzugsweise in Form mindestens eines Cytokins,
gebildet wird.

2. Verfahren nach Anspruch 1, dadurch gekennzeichnet, daß als Strukturkomponente ein makromolekulares Gel und/oder Netzwerk, vorzugsweise aus Proteoglycanen und Collagenen verwendet wird.

3. Verfahren nach Anspruch 1 oder 2, dadurch gekennzeichnet, daß die Strukturkomponente metallische und/oder keramische und/oder glasartige und/oder polymere und/oder fetthaltige Trägermaterialien in solider und/oder poröser und/oder membranöser und/oder mizellarer und/oder plastisch verformbarer und/oder viskoser und/oder flüssiger Form aufweist.

4. Verfahren nach einem der Ansprüche 1 bis 3, dadurch gekennzeichnet, daß die jeweilige Rekrutierungskomponente zur Gruppe der chemotaktischen Peptide, Proteine und/oder der Arachidonsäuremetabolite gehört oder eine andere gleichwirkende chemische Struktur aufweist.

5. Verfahren nach einem der Ansprüche 1 bis 4, dadurch gekennzeichnet, daß als jeweilige Adhäsionskomponente ein Protein vom Typ des Fibronectin, Tenascin, Cytotactin, Laminin, Chondronectin, Collagen Typ IV, V, VII, N-CAM, L-CAM oder Integrin, eine Kombination dieser Proteine oder ein anderes gleichwirkendes Adhäsin verwendet wird.

6. Verfahren nach einem der Ansprüche 1 bis 5, dadurch gekennzeichnet, daß die jeweilige Wachstums- und/oder Maturationskomponente ausgewählt wird aus der Gruppe der Kolonie stimulierenden Faktoren (CSF), der Interleukine (IL), der Insulin ähnlichen Wachstumsfaktoren (IGF), der Prostaglandine (PG), der Leukotriene (LT), der Transformierenden Wachstumsfaktoren (TGF), der Fibroblasten Wachstumsfaktoren (FGF), der Interferone (IFN), der epidermalen Wachstumsfaktoren (EGF), der von Knochen abstammenden Wachstumsfaktoren (BDGF), der von Endothelien abstammenden Wachstumsfaktoren (EDGF), der von Thrombocyten abstammenden Wachstumsfaktoren (PDGF), einer Kombination derartiger Faktoren oder einem anderen gleichwirkenden Cytokin.

7. Verfahren nach einem der Ansprüche 1 bis 6, dadurch gekennzeichnet, daß die jeweilige Wachstums- und/oder Maturationskomponente Faktoren zum beschleunigten Einwachsen von Blutgefäßen, wie Angiogenese-Faktoren und/oder von Nerven, wie Nerven-Wachstums-Faktoren, in das herzustellende biologische Teil aufweist.

8. Verfahren nach einem der Ansprüche 1 bis 7, dadurch gekennzeichnet, daß die Abbaugeschwindigkeit des Wirkstoffkomplexes durch den unterschiedlichen Vernetzungsgrad der Strukturkomponente selbst und/oder den Zusatz von (Enzym)inhibitoren und/oder immunsuppressiven und/oder entzündungshemmenden Wirkstoffen gesteuert wird.

9. Verfahren nach einem der Ansprüche 1 bis 8, dadurch gekennzeichnet, daß der Wirkstoffkomplex zusammen mit Zielzellen ein biologisches Teil, zumindest im Aufbaustadium desselben, bildet.

10. Verfahren nach einem der Ansprüche 1 bis 9, dadurch gekennzeichnet, daß der Wirkstoffkomplex durch Kopräzipitation der verschiedenen Komponenten oder durch Lyophilisation oder durch kovalente Verknüpfung der einzelnen Komponenten miteinander hergestellt wird.

11. Verfahren nach einem der Ansprüche 1 bis 10, bei dem der gemäß einem der Ansprüche 1 bis 10 hergestellte Wirkstoffkomplex zur Behandlung von Krankheiten, die durch das Fehlen des biologischen Teils oder dessen Fehlfunktion bedingt sind, verwendet wird.

## Claims (Claims for the following Contracting State(s): AT, BE, CH, LI, DE, DK, FR, GB, IT, LU, NL, SE)

1. An active compound complex for the production of biological parts, in particular of organs for living beings, having the following components, which are different from one another and are specifically suited to the biological part to be produced in each case, in the form of
- at least one structural component based on extracellular material specifically suited to the cells of the biological part to be produced in each case,
- at least one recruiting component,
- at least one adhesion component,
- at least one growth and/or maturation component, preferably in the form of at least one cytokine.

2. An active compound complex according to claim 1, characterized in that the structural component is a macromolecular gel and/or network, preferably made of proteoglycans and collagens.

3. An active compound complex according to either of claims 1 and 2, characterized in that the structural component contains metallic and/or ceramic and/or glassy and/or polymeric and/or fat-containing support materials in solid and/or porous and/or membranous and/or micellar and/or plastically deformable and/or viscous and/or liquid form.

4. An active compound complex according to any one of claims 1 to 3, characterized in that the respective recruiting component belongs to the group consisting of the chemotactic peptides, proteins and/or arachidonic acid metabolites or has another identically acting chemical structure.

5. An active compound complex according to any one of claims 1 to 4, characterized in that the respective adhesion component is a protein of the fibronectin, tenascin, cytotactin, laminin, chondronectin, collagen Type IV, V, VII, N-CAN, L-CAM or integrin type, a combination of these proteins or another identically acting adhesin.

6. An active compound complex according to any one of claims 1 to 5, characterized in that the respective growth and/or maturation component belongs to the group consisting of the colony-stimulating factors (CSF), the interleukins (IL), the insulin-like growth factors (IGF), the prostaglandins (PG), the leukotrienes (LT), the transforming growth factors (TGF), the fibroblast growth factors (FGF), the interferons (IFN), the epidermal growth factors (EGF), the bone-derived growth factors (BDGF), the endothelium-derived growth factors (EDGF), the platelet-derived growth factors (PDGF), a combination of factors of this type or another identically acting cytokine.

7. An active compound complex according to any one of claims 1 to 6, characterized in that the respective growth and/or maturation component contains factors for the accelerated growth of blood vessels, such as angiogenesis factors, and/or of nerves, such as nerve growth factors, in the biological part to be produced.

8. An active compound complex according to any one of claims 1 to 7, characterized in that its breakdown rate is controllable by means of the different degree of crosslinking of the structural component itself and/or the addition of (enzyme) inhibitors and/or immunosuppressant and/or anti-inflammatory active compounds.

9. An active compound complex according to any one of claims 1 to 8, characterized in that this, together with target cells, forms a biological part, at least in the construction stage thereof.

10. A process for producing the active compound complex according to any one of claims 1 to 9, characterized in that this can be prepared by coprecipitation of the various components or by lyophilization or by covalent linkage of the individual components to one another.

11. The use of the active compound complex according to any one of claims 1 to 9 for the production of a biological part for the treatment of diseases which are caused by the absence of the biological part or its dysfunction.

## Claims (Claims for the following Contracting State(s): ES, GR)

1. A process for the preparation of an active compound complex for the production of biological parts, in particular of organs for living beings, which is formed with the following components, which are different from one another and are specifically suited to the biological part to be produced in each case, in the form of
- at least one structural component based on extracellular material specifically suited to the cells of the biological part to be produced in each case,
- at least one recruiting component,
- at least one adhesion component,
- at least one growth and/or maturation component,
preferably in the form of at least one cytokine.

2. A process according to claim 1, characterized in that the structural component used is a macromolecular gel and/or network, preferably made of proteoglycans and collagens.

3. A process according to either of claims 1 and 2, characterized in that the structural component contains metallic and/or ceramic and/or glassy and/or polymeric and/or fat-containing support materials in solid and/or porous and/or membranous and/or micellar and/or plastically deformable and/or viscous and/or liquid form.

4. A process according to any one of claims 1 to 3, characterized in that the respective recruiting component belongs to the group consisting of the chemotactic peptides, proteins and/or the arachidonic acid metabolites or has another identically acting chemical structure.

5. A process according to any one of claims 1 to 4, characterized in that the respective adhesion component used is a protein of the fibronectin, tenascin, cytotactin, laminin, chondronectin, collagen Type IV, V, VII, N-CAM, L-CAM or integrin type, a combination of these proteins or another identically acting adhesin.

6. A process according to any one of claims 1 to 5, characterized in that the respective growth and/or maturation component is selected from the group consisting of the colony-stimulating factors (CSF), the interleukins (IL), the insulin-like growth factors (IGF), the prostaglandins (PG), the leukotrienes (LT), the transforming growth factors (TGF), the fibroblast growth factors (FGF), the interferons (IFN), the epidermal growth factors (EGF), the bone-derived growth factors (BDGF), the endothelium-derived growth factors (EDGF), the platelet-derived growth factors (PDGF), a combination of factors of this type or another identically acting cytokine.

7. A process according to any one of claims 1 to 6, characterized in that the respective growth and/or maturation component contains factors for the accelerated growth of blood vessels, such as angiogenesis factors, and/or of nerves, such as nerve growth factors, in the biological part to be produced.

8. A process according to any one of claims 1 to 7, characterized in that the breakdown rate of the active compound complex is controlled by the different degree of crosslinking of the structural component itself and/or the addition of (enzyme) inhibitors and/or immunosuppressant and/or anti-inflammatory active compounds.

9. A process according to any one of claims 1 to 8, characterized in that the active compound complex, together with target cells, forms a biological part, at least in the construction stage thereof.

10. A process according to any one of claims 1 to 9, characterized in that the active compound complex is prepared by coprecipitation of the various components or by lyophilization or by covalent linkage of the individual components to one another.

11. A process according to any one of claims 1 to 10, in which the active compound complex prepared according to any one of claims 1 to 10 is used for the treatment of diseases which are caused by the absence of the biological part or its dysfunction.

## Revendications (Revendications pour l'(les) Etat(s) contractant(s) suivant(s): AT, BE, CH, LI, DE, DK, FR, GB, IT, LN, NL, SE)

1. Complexe de substances actives pour la préparation d'éléments biologiques, en particulier d'organes d'êtres vivants, comprenant les composantes suivantes, différentes les unes des autres et choisies spécifiquement en fonction de l'élément biologique à préparer, qui sont
- au moins une composante structurale à base de matériau extracellulaire correspondant spécifiquement aux cellules de l'élément biologique à préparer,
- au moins une composante de recrutement,
- au moins une composante d'adhésion,
- au moins une composante de croissance et/ou de maturation, de préférence au moins une cytokine.

2. Complexe de substances actives selon la revendication 1, caractérisé par le fait que la composante structurale est un gel et/ou un réseau macromoléculaire, de préférence formé par des protéoglycanes et du collagène.

3. Complexe de substances actives selon la revendication 1 ou 2, caractérisé par le fait que la composante structurale comprend des matériaux support métalliques et/ou céramiques et/ou vitreux et/ou polymères et/ou contenant des graisses sous forme solide et/ou poreuse et/ou membranaire et/ou micellaire et/ou plastique et/ou visqueuse et/ou liquide.

4. Complexe de substances actives selon une des revendications 1 à 3, caractérisé par le fait que ladite composante de recrutement appartient au groupe des peptides chimiotactiques, des protéines chimiotactiques et/ou des métabolites de l'acide arachidonique ou présente une structure chimique ayant une action similaire.

5. Complexe de substances actives selon une des revendications 1 à 4, caractérisé par le fait que ladite composante d'adhésion est une protéine de type fibronectine, ténascine, cytotactine, laminine, chondronectine, collagène de type IV, V. VII, N-CAM, L-CAM ou intégrine, une combinaison de ces protéines ou une adhésine ayant une action similaire.

6. Complexe de substances actives selon l'une des revendications 1 à 5, caractérisé par le fait que ladite composante de croissance et/ou de maturation est choisie dans le groupe formé par les facteurs stimulateurs de colonies (CSF), les interleukines (IL), les facteurs de croissance ressemblant à l'insuline (IGF), les prostaglandines (PG), les leukotriènes (LT), les facteurs de croissance transformants (TGF) les facteurs de croissance des fibroblastes (FGF), les interférones (IFN), les facteurs de croissance de l'épiderme (EGF), les facteurs de croissance dérivés des os (BDGF), les facteurs de croissance dérivés de l'endothélium (EDGF), les facteurs de croissance dérivés des thrombocytes (PDGF), ou est une combinaison de tels facteurs ou une cytokine ayant une action similaire.

7. Complexe de substances actives selon une des revendications 1 à 6, caractérisé par le fait que ladite composante de croissance et/ou de maturation comprend des facteurs pour accélérer la pénétration de l'élément biologique à préparer par des vaisseaux sanguins, tels que des facteurs de l'angiogenèse, et/ou par des nerfs tels que les facteurs de croissance des nerfs.

8. Complexe de substances actives selon une des revendications 1 à 7, caractérisé par le fait que l'on peut faire varier sa vitesse de dégradation par l'intermédiaire du taux de réticulation de la composante structurale elle-même et/ou par addition d'inhibiteurs (d'enzymes) et/ou de substances immunosuppressives et/ou anti-inflammatoires.

9. Complexe de substances actives selon une quelconque des revendications 1 à 8, caractérisé par le fait qu'il forme, en conjonction avec des cellules cibles, un élément biologique, au moins au stade de la formation.

10. Procédé de préparation d'un complexe de substances actives selon une des revendications 1 à 9, caractérisé par le fait que l'on peut l'obtenir par coprécipitation des différentes composantes ou par lyophilisation ou par liaison covalente des différentes composantes les unes aux autres.

11. Utilisation du complexe de substances actives selon une des revendications 1 à 9 pour la préparation d'un élément biologique destiné au traitement de maladies dues à l'absence de l'élément biologique ou à un dysfonctionnement de celui-ci.

## Revendications (Revendications pour l'(les) Etat(s) contractant(s) suivant(s): ES, GR)

1. Procédé de préparation d'un complexe de substances actives pour la préparation d'éléments biologiques, en particulier d'organes d'êtres vivants, formé à partir des composantes suivantes, différentes les unes des autres et choisies spécifiquement en fonction de l'élément biologique à préparer, qui sont
- au moins une composante structurale à base de matériau extracellulaire correspondant spécifiquement aux cellules de l'élément biologique à préparer,
- au moins une composante de recrutement,
- au moins une composante d'adhésion,
- au moins une composante de croissance et/ou de maturation, de préférence au moins une cytokine.

2. Procédé selon la revendication 1, caractérisé par le fait que l'on utilise comme composante structurale un gel et/ou un réseau macromoléculaire, de préférence formé par des protéoglycanes et du collagène.

3. Procédé selon selon la revendication 1 ou 2, caractérisé par le fait que la composante structurale comprend des matériaux support métalliques et/ou céramiques et/ou vitreux et/ou polymères et/ou contenant des graisses sous forme solide et/ou poreuse et/ou membranaire et/ou micellaire et/ou plastique et/ou visqueuse et/ou liquide.

4. Procédé selon une des revendications 1 à 3, caractérisé par le fait que ladite composante de recrutement appartient au groupe des peptides chimiotactiques, des protéines chimiotactiques et/ou des métabolites de l'acide arachidonique ou présente une structure chimique ayant une action similaire.

5. Procédé selon une des revendications 1 à 4, caractérisé par le fait que ladite composante d'adhésion est une protéine de type fibronectine, ténascine, cytotactine, laminine, chondronectine, collagène de type IV, V, VII, N-CAM, L-CAM ou intégrine, une combinaison de ces protéines ou une adhésine ayant une action similaire.

6. Procédé selon une des revendications 1 à 5, caractérisé par le fait que la composante de croissance et/ou de maturation appartient au groupe formé par les facteurs stimulateurs de colonies (CSF), les interleukines (IL), les facteurs de croissance ressemblant à l'insuline (IGF), les prostaglandines (PG), les leukotriènes (LT), les facteurs de croissance transformants (TGF) les facteurs de croissance des fibroblastes (FGF), les interférones (IFN), les facteurs de croissance de l'épiderme (EGF), les facteurs de croissance dérivés des os (BDGF), les facteurs de croissance dérivés de l'endothélium (EDGF), les facteurs de croissance dérivés des thrombocytes (PDGF), ou est une combinaison de tels facteurs ou une cytokine ayant une action similaire.

7. Procédé selon une des revendications 1 à 6, caractérisé par le fait que ladite composante de croissance et/ou de maturation comprend des facteurs pour accélérer la pénétration de l'élément biologique à préparer par des vaisseaux sanguins, tels que des facteurs de l'angiogenèse, et/ou par des nerfs tels que les facteurs de croissance des nerfs.

8. Procédé selon une des revendications 1 à 7, caractérisé par le fait que l'on peut faire varier sa vitesse de dégradation par l'intermédiaire du taux de réticulation de la composante structurale elle-même et/ou par addition d'inhibiteurs (d'enzymes) et/ou de substances immuno-suppressives et/ou anti-inflammatoires.

9. Procédé selon une des revendications 1 à 8, caractérisé par le fait que le complexe de substances actives forme, en conjonction avec des cellules cibles, un élément biologique, au moins au stade de la formation.

10. Procédé selon une des revendications 1 à 9, caractérisé par le fait que le complexe de substances actives est préparé par coprécipitation des différentes composantes ou par lyophilisation ou par liaison covalente des différents composants les unes aux autres.

11. Procédé selon une des revendications 1 à 10 dans lequel on utilise le complexe de substances actives préparé selon une des revendications 1 à 10 pour le traitement de maladies dues à l'absence de l'élément biologique ou à un dysfonctionnement de celui-ci.
